# NOUVEAU FASCICULE DE BREVET EUROPEEN

(11) **EP 0 987 015 B2**
(45) Date de publication et mention de la décision concernant l'opposition: **11.03.2009**
(45) Mention de la délivrance du brevet: 31.05.2006
(21) Numéro de dépôt: 99402078.2
(22) Date de dépôt: 17.08.1999
(51) Int. Cl.: A61K 8/06, A61K 8/73, A61K 8/81, A61Q 1/14, A61Q 19/00

(54) **Emulsion comprenant un composé épaississant hydrophile et un alkléther de polysaccharide, compositions comprenant ladite émulsion, et utilisations**
Verdickungsmittel und Alkylether eines Polysaccharides enthaltende hydrophile Emulsion, diese enthaltende Zusammensetzungen und ihre Verwendung
Emulsion containing an hydrophile thickening agent and an alkylether of polysaccharide, compositions containing them and their uses

(30) Priorité: 16.09.1998 FR 9811576
(43) Date de publication de la demande: 22.03.2000
(73) Titulaire: L'ORÉAL, 75008 Paris (FR)
(72) Inventeur: Lorant, Raluca, 94320 Thiais (FR)
(74) Mandataire: Le Coupanec, Pascale A.M.P.

(56) Documents cités:
- EP-A- 0 412 705
- EP-A- 0 795 321
- EP-A- 0 795 322
- EP-A- 0 795 323
- EP-A2- 0 804 923
- EP-A2- 0 804 924
- EP-B1- 0 412 705
- EP-B1- 0 795 320

## Description

La présente invention a trait à une composition se présentant sous la forme d'une émulsion, susceptible d'être utilisée dans les domaines cosmétique et dermatologique, notamment pour le soin ou le traitement de la peau du corps ou du visage, plus particulièrement pour le soin ou le traitement des peaux sèches et/ou sensibles. L'invention concerne également une composition cosmétique ou dermatologique comprenant une telle émulsion.

Les compositions cosmétiques ou dermatologiques actuelles se présentent le plus souvent sous la forme d'émulsion du type huile-dans-eau (c'est à dire un support constitué d'une phase continue dispersante aqueuse et d'une phase discontinue dispersée huileuse) ou d'émulsion du type eau-dans-huile (c'est à dire un support constitué d'une phase continue dispersante grasse et d'une phase discontinue dispersée aqueuse).
Les émulsions eau-dans-huile comportent donc une phase continue huileuse et permettent de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.
Les émulsions huile-dans-eau, quant à elles, apportent sur la peau à l'application un toucher plus doux, moins gras et plus léger que les émulsions eau-dans-huile.

Les émulsions sont stabilisées généralement par incorporation de tensioactifs émulsionnants du type huile-dans-eau (H/E) ou du type eau-dans-huile (E/H) qui, grâce à leur structure amphiphile, se placent à l'interface huile/eau et stabilisent ainsi les gouttelettes dispersées. Il est généralement nécessaire d'introduire ces tensioactifs en une quantité importante, pouvant aller jusqu'à 10 % en poids par rapport au poids total de l'émulsion, pour obtenir une stabilité adéquate.
Or, ces tensioactifs amphiphiles, utilisés en grande quantité, peuvent se montrer irritants pour la peau, les yeux et/ou le cuir chevelu des utilisateurs. De plus, leur présence à de fortes concentrations peut conduire à des effets non cosmétiques tels qu'un toucher rêche, collant et/ou poisseux, ou une composition finale compacte et lourde. D'autre part, les tensioactifs doivent être choisis en fonction de la polarité des huiles et ne sont donc compatibles qu'avec un nombre limité d'huiles, limitant ainsi la variété des formulations.

Les formulateurs d'émulsions recherchent constamment à réduire la teneur en tensioactif afin d'améliorer l'innocuité des émulsions vis à vis de la peau, des yeux et/ou du cuir chevelu et améliorer leurs propriétés cosmétiques. La principale difficulté à laquelle ils sont généralement confrontés est d'obtenir des émulsions stables.

Il a ainsi été proposé, par la demande WO96/37180, une composition susceptible d'applications dans les domaines pharmaceutique et/ou cosmétique, se présentant sous forme de 'pseudo-émulsion' et dépourvue de tensioactif. La composition comprend, d'une part, en phase aqueuse, un agent gélifiant choisi notamment parmi les polyoses ou les polymères acryliques, et d'autre part, en phase grasse, un facteur de consistance choisi parmi les corps gras cireux, et notamment les esters de glycérol. Le facteur de consistance présent dans la phase grasse est une substance semi-solide à 25°C et a un point de fusion supérieur à 50°C; il est solubilisé à chaud dans la phase grasse puis retrouve sa consistance semi-solide initiale à froid, conférant une certaine consistance et une certaine viscosité à ladite phase grasse. La composition ainsi obtenue présente une structure microscopique différente de celle d'une émulsion.
On a toutefois constaté qu'au delà d'une certaine quantité de phase grasse, ladite pseudo-émulsion perdait en stabilité, à la chaleur et/ou dans le temps. Ceci est en particulier vrai pour des taux de phase grasse supérieurs à 20% en poids par rapport au poids total de la pseudo-émulsion. Or, un taux de phase grasse important, dans une émulsion H/E, peut se révéler très intéressant, notamment pour les compositions cosmétiques destinées au soin des peaux sèches. De plus, les propriétés cosmétiques de cette pseudo-émulsion ne sont pas adéquates: sa texture est hétérogène, son toucher gras, et on constate une mauvaise prise sur le doigt.
Ainsi, il subsiste toujours le besoin de disposer d'une émulsion H/E qui soit stable au cours du temps bien qu'elle ne contienne pas de tensioactif, et qui puisse comprendre une quantité importante de phase grasse sans perdre sa stabilité.

La présente invention a pour but de pallier ce besoin et de proposer une émulsion, notamment une émulsion H/E ne présentant pas les inconvénients mentionnés ci-dessus, et qui soit stable tout en comprenant une quantité réduite de tensioactif, et qui puisse supporter une quantité importante de phase grasse.
Ainsi, la demanderesse a trouvé de manière surprenante qu'il était possible d'obtenir une émulsion ayant de bonnes propriétés cosmétiques et une bonne stabilité en utilisant une association particulière d'épaississants, de la phase huileuse et de la phase aqueuse.

La présente invention a donc pour objet une émulsion comprenant une phase aqueuse et une phase grasse, comprenant en outre au moins un composé épaississant hydrophile, au moins un alkyléther de polysaccharide formé de deux motifs comportant au moins deux cycles osidiques différents, chaque motif comportant au moins un groupe hydroxyle substitué par une chaîne alkyle hydrocarbonée saturée, et au moins un milieu solvant de l'alkyléther de polysaccharide, ledit milieu solvant représentant de 75 à 99,9% en poids du poids total de la phase huileuse de l'émulsion.

L'invention a également pour objet une composition cosmétique ou dermatologique comprenant, dans un milieu cosmétiquement ou dermatologiquement acceptable, une telle émulsion.

L'invention a encore pour objet l'utilisation d'une telle émulsion pour le traitement cosmétique de la peau, des cheveux, des cils, des sourcils, des ongles, des muqueuses, du cuir chevelu; en particulier pour le soin du visage et/ou du corps, le maquillage du visage et/ou du corps, le démaquillage; la protection solaire; et/ou pour la fabrication d'une composition dermatologique destinée au traitement de la peau, des cheveux, des cils, des sourcils, des ongles, du cuir chevelu et/ou des muqueuses.

Un autre objet de l'invention est un procédé de traitement non thérapeutique de la peau, notamment des peaux sèches et/ou sensibles, des cheveux, des cils, des sourcils, des ongles, des muqueuses, du cuir chevelu, consistant à appliquer sur le support une émulsion telle que définie ci-dessus.

Encore un objet de l'invention est l'utilisation d'un composé épaississant hydrophile et d'un alkyléther de polysaccharide tel que ci-dessus défini, pour stabiliser une émulsion comprenant au moins un milieu solvant dudit alkyléther de polysaccharide, en particulier une émulsion huile-dans-eau et de préférence une émulsion ne comprenant pas de tensioactif, représentant de 75% à 99,9% en poids du poids total de la phase huileuse de l'émulsion.

On a constaté que l'émulsion obtenue selon l'invention reste stable dans le temps à température ambiante ou à des températures plus élevées, malgré la faible quantité voire l'absence de tensioactif.
De plus, la phase grasse se disperse parfaitement dans la phase aqueuse et permet l'obtention d'une composition homogène, ayant la structure microscopique d'une émulsion.
L'émulsion obtenue est facile à appliquer sur la peau, les muqueuses, le cuir chevelu, les cheveux, les cils, les sourcils, les ongles.
Les textures obtenues sont particulièrement originales: les émulsions sont crémeuses et onctueuses, et n'ont absolument pas l'aspect gélifié, voire gélatineux, de certaines émulsions de l'art antérieur dont la phase aqueuse externe est gélifiée.
Le toucher cosmétique sur la peau est également apprécié: à l'application, l'émulsion procure une sensation de fraîcheur et de confort, tout en étant riche et nourrissante; elle est douce et confortable et pas du tout collante.
L'émulsion ainsi obtenue est particulièrement adaptée pour le soin et le traitement des peaux sèches et/ou sensibles, de par la possibilité de la présence d'une quantité importante de corps gras apportant soin et confort, et de l'absence de tensioactif irritant.
De plus, les émulsions selon l'invention ne nécessitent pas de mode opératoire lourd et coûteux tel qu'une homogénéisation à haute pression, mais peuvent être préparées selon un mode opératoire classique.

L'émulsion selon l'invention se présente, de manière préférentielle, sous la forme d'une émulsion huile-dans-eau, comprenant une phase grasse ou huileuse interne dispersée dans une phase externe aqueuse.

La phase aqueuse peut comprendre de l'eau et/ou une eau thermale et/ou une eau de source et/ou une eau minérale et/ou une eau florale.
L'émulsion comprend par ailleurs au moins un composé épaississant hydrophile, qui peut être choisi parmi tous les épaississants hydrophiles connus de l'homme du métier. En particulier, on peut citer les composés suivants :
- les polymères synthétiques,
- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxy-méthylcellulose,
- le silicate de magnésium et d'aluminium;
- les épaississants inorganiques tels que les smectites, les hectorites modifiées ou non (BENTONE, LAPONITE par exemple),
- leurs mélanges.

Parmi les polymères synthétiques, on peut citer :
(A) les acides polyacryliques et notamment :
   (i) les polymères poly(méth)acrylates de glycéryl tels que l'HISPAGEL ou le LUBRAGEL des sociétés HISPANO QUIMICA ou GARDIAN,
   (ii) les homopolymères et les copolymères d'acide acrylique, éventuellement réticulés, ou un de leurs sels, tels que ceux commercialisés sous le nom 'CARBOPOL' par la société GOODRICH;
(B) les polymères à base de polyacrylamide et notamment :
   (i) le produit commercialisé sous le nom de SEPIGEL 305 par SEPPIC, et qui est constitué d'une émulsion H/E comprenant 35-45% en poids de copolymère réticulé d'acrylamide/acide 2-acrylamido 2-méthylpropane sulfonique neutralisé, 15-25% en poids d'hydrocarbures isoparaffiniques, 3-8% en poids de lauryléther de polyéthylèneglycol 70E, et d'eau;
   (ii) les copolymères acrylate/octyl-acrylamide tels que le DERMACRYL de National Starch;
   (iii) les polymères réticulés d'acrylamide et de chlorure de méthacryloyloxy éthyltriméthylammonium tels que le SALCARE SC92 de ALLIED COLLOIDS;
   (iv) les polymères réticulés d'acrylamide et d'acrylate d'ammonium tels que le PAS 5161 ou BOZEPOL C de HOECHST;
   (v) les polymères poly(acide 2-acrylamido 2-méthylpropane sulfonique) réticulés et pratiquement ou totalement neutralisés.
(C) les copolymères constitués d'une fraction majoritaire de monomère acide carboxylique monooléfiniquement insaturé en C₃-C₅ ou de son anhydride, et d'une fraction minoritaire de monomère ester à chaîne grasse d'acide acrylique, ces copolymères étant éventuellement réticulés.

Les polymères poly(acide 2-acrylamido 2-méthylpropane sulfonique) réticulés et pratiquement ou totalement neutralisés, susceptibles d'être utilisés dans le cadre de l'invention, sont hydrosolubles ou gonflables dans l'eau. Ils sont en général caractérisés par le fait qu'ils comprennent, distribués de façon aléatoire :
a) de 90 à 99,9% en poids de motifs de formule générale (1) suivante : 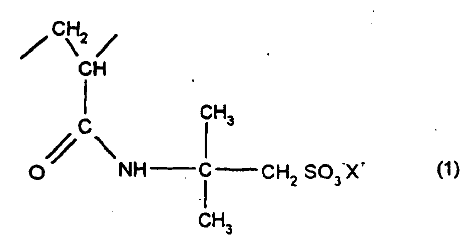 dans laquelle X⁺ désigne un cation ou un mélange de cations, au plus 10% mol des cations X⁺ pouvant être des protons H⁺ ;
b) de 0,01 à 10% en poids de motifs réticulants provenant d'au moins un monomère ayant au moins deux doubles liaisons oléfiniques ; les proportions en poids étant définis par rapport au poids total du polymère.
De façon préférentielle, ils comprennent de 98 à 99,5 % en poids de motifs de formule (1) et de 0,2 à 2 % en poids de motifs réticulants.
X⁺ représente un cation ou un mélange de cations choisis en particulier parmi un proton, un cation de métal alcalin, un cation équivalent de celui d'un métal alcalino-terreux ou l'ion ammonium. Plus particulièrement, 90 à 100% mole des cations sont des cations NH₄⁺ et 0 à 10% mole sont des protons (H⁺).
Les monomères de réticulation ayant au moins deux doubles liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.
Les monomères de réticulation ayant au moins deux doubles liaisons oléfiniques sont plus particulièrement choisis parmi ceux répondant à la formule générale (2) suivante : 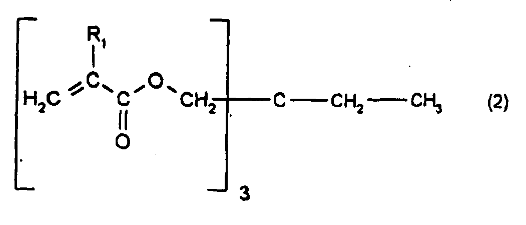 dans laquelle R₁ désigne un atome d'hydrogène ou un alkyle en C₁-C₄ et plus particulièrement méthyle (triméthylol propane triacrylate).
De manière préférée, on choisit ces polymères réticulés parmi ceux présentant une viscosité mesurée au viscosimètre BROOKFIELD, mobile 4, à une vitesse de rotation de 100 tours/minutes dans une solution d'eau à 2 % et à 25 °C supérieure ou égale à 1000 centipoises et plus préférentiellement allant de 5000 à 40000 centipoises et plus particulièrement de 6500 à 35000 centipoises.
Ces polymères sont notamment décrits dans la demande EP815844.
Il est à noter que cette demande EP815844 est relative à une composition cosmétique ou dermatologique sous forme d'émulsion huile-dans-eau, qui est stable tout en ne contenant pas de tensioactif, et qui comprend par ailleurs un polymère poly(acide 2-acrylamido 2-méthylpropane sulfonique) réticulé et neutralisé à au moins 90%. Ce polymère est ajouté à la phase aqueuse, qu'il épaissit. Toutefois, dans la composition ainsi obtenue, la phase grasse ne peut représenter que jusqu'à environ 12-15% en poids de l'émulsion. Au-delà de cette quantité de phase grasse, la composition perd en stabilité.

Les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique, susceptibles d'être utilisés dans le cadre de la présente invention, éventuellement réticulés, peuvent être préparés en polymérisant une quantité prépondérante de monomère carboxylique monooléfiniquement insaturé ou de son anhydride, avec une quantité plus faible de monomère ester acrylique à chaîne grasse. La quantité de monomère carboxylique ou de son anhydride, est de préférence comprise entre 80 et 98% en poids et plus particulièrement entre 90 et 98% en poids; l'ester acrylique est de préférence présent dans des quantités comprises entre 2 et 20% en poids et plus particulièrement entre 2 et 10% en poids; les pourcentages sont calculés par rapport au poids des deux monomères.
Les monomères carboxyliques préférentiels sont choisis parmi ceux répondant à la formule : CH₂=CR-COOH
dans laquelle R désigne hydrogène, halogène, hydroxyle, un groupe lactone, un groupe lactame, un groupe cyanogène (-CN), un groupe alkyle monovalent, un groupe aryle, un groupe alkylaryle, un groupe aralkyle ou un groupe cycloaliphatique.

Les monomères carboxyliques particulièrement préférés, sont choisis parmi l'acide acrylique, l'acide méthacrylique, l'anhydride maléique, et leurs mélanges.
Les monomères esters acryliques à chaîne grasse sont préférentiellement choisis parmi ceux répondant à la formule : CH₂=CR¹-COOR²
dans laquelle R¹ est choisi dans le groupe formé par hydrogène, méthyle et éthyle et R² est un groupe alkyle en C₈-C₃₀, un groupe oxyalkylène en C₈-C₃₀, un groupe carbonyloxyalkylène en C₈-C₃₀.
Les monomères esters particulièrement préférés sont ceux pour lesquels R¹ est hydrogène ou méthyle, et/ou ceux pour lesquels R² est un groupe alkyle en C₁₀₋C₂₂. On peut notamment citer les acrylates et méthacrylates de décyle, de lauryle, de stéaryle, de béhényle ou de mélissyle.
Certains de ces copolymères sont notamment décrits dans la demande EP-A-0268164 et sont obtenus selon les méthodes de préparation décrites dans ce même document.
On peut citer plus particulièrement les copolymères vendus sous le nom PEMULEN par la Société GOODRICH, et notamment le copolymère acrylate/C₁₀₋C₃₀-alkylacrylate tel que le produit PEMULEN TR 2.

Dans le cadre de la présente invention, on peut, bien évidemment, utiliser un mélange de plusieurs épaississants hydrophiles tels que ci-dessus définis. Lesdits épaississants hydrophiles peuvent être présents dans les compositions cosmétiques ou dermatologiques de l'invention dans des concentrations allant de préférence de 0,05-10 % en poids par rapport au poids total de la composition et plus préférentiellement de 0,5-4 % en poids.
De préférence, la viscosité de la phase aqueuse est de l'ordre de 30 000 à 80 000 centipoises (30-80 Pa.s) mesurée au viscosimètre Brookfield, aiguille 7 à une vitesse de 20 tours/minute.

L'émulsion selon l'invention comprend par ailleurs une phase huileuse qui comprend au moins un alkyléther de polysaccharide formé de deux motifs comportant au moins deux cycles osidiques différents, chaque motif comportant au moins un groupe hydroxyle substitué par une chaîne alkyle hydrocarbonée saturée.
Par "chaîne alkyle hydrocarbonée", on entend une chaîne linéaire ou ramifiée, comportant de 1 à 24, de préférence de 1 à 10, mieux de 1 à 6 et plus spécialement de 1 à 3 atomes de carbone. En particulier, la chaîne alkyle est choisie parmi les chaînes saturées et notamment méthyle, éthyle, éthényle, n-propyle, propényle, isopropyle, n-butyle, isobutyle, tertiobutyle, n-pentyle.
Les cycles osidiques sont notamment choisis parmi le mannose, le galactose, le glucose, le furanose, le rhamnose, l'arabinose.
Ces alkyléthers peuvent être fabriqués comme décrits dans les documents EP-A-281 360, EP-A-708 114
De préférence, l'alkyléther de polysaccharide a un poids moléculaire moyen en poids supérieur à 100 000, et de préférence supérieur à 200 000. Ce poids moléculaire peut aller jusqu'à 1 million. Cet alkyléther peut comporter de un à six et mieux de deux à quatre groupes hydroxyle par motif, substitués par une chaîne alkyle hydrocarbonée saturée ou non.

Selon un mode préféré de réalisation de l'invention, l'alkyléther de polysaccharide est un alkyléther d'une gomme et plus particulièrement d'une gomme globalement non ionique, c'est-à-dire comportant peu ou pas de groupe ionique.
Comme gommes appropriées, on peut citer par exemple la gomme de guar dont le motif comprend un galactose et un mannose; la gomme de caroube dont le motif comprend un galactose et un mannose; la gomme de karaya qui est un mélange complexe de rhamnose, galactose et acide galacturonique; la gomme adragante qui est un mélange complexe d'arabinose, galactose et acide galacturonique.
De préférence, l'alkyléther de polysaccharide est un dérivé de gomme de guar. Ainsi, avantageusement l'alkyéther est un galactomannane alkylé de chaîne alkyle en C₁ à C₆ et mieux en C₁ à C₃ et plus particulièrement le guar éthylé ayant un degré de substitution de 2 à 3 et notamment d'environ 2,5 à 2,9, tel que décrit dans les documents RD 9537807 (octobre 1995) et EP-A-708114. Parmi les produits commerciaux, on peut citer les gommes vendues par la société Aqualon sous les noms N-HANCE-AG 200® et N-HANCE AG 50® .
Dans le cadre de la présente invention, on peut bien évidemment utiliser un mélange de plusieurs alkyléthers de polysaccharide tels que ci-dessus définis.
Lesdits alkyléthers de polysaccharide peuvent être présents dans les émulsions et/ou compositions selon l'invention dans des concentrations allant de préférence de 0,1-10 % en poids par rapport au poids total de la composition et plus préférentiellement de 0,5-2 % en poids.
Avantageusement, l'alkyléther de polysaccharide peut être présent en une quantité telle que le rapport (en poids) de la quantité d'huile sur la quantité d'alkyléther de polysaccharide est choisi dans la gamme allant de 5 à 1000.
De préférence, la viscosité de la phase huileuse est de l'ordre de 6 000 à 20 000 centipoises (6-20 Pa.s) mesurée au viscosimètre Brookfield, aiguille 5 à une vitesse de 20 tours/minute.

La phase grasse liquide de l'émulsion selon l'invention comprend au moins un milieu solvant de l'alkyléther de polysaccharide qui peut être une huile. Par huile, on entend toute matière grasse liquide à température ambiante (25°C).
Parmi les huiles utilisables comme milieu solvant de l'alkyléther de polysaccharide selon l'invention, on peut citer par exemple :
- les huiles d'origine végétale comme les triglycérides liquides, par exemple les huiles de tournesol, de maïs, de soja, de jojoba, de courge, de pépins de raisin, de sésame, de noisette, d'abricot, de macadamia, de ricin; les triglycérides des acides caprylique/caprique comme ceux vendus par la société STEARINERIES DUBOIS ou ceux vendus sous les dénominations MIGLYOL 810, 812 et 818® par la société DYNAMIT NOBEL.
- les huiles d'origine animale telle que la lanoline,
- les huiles d'origine minérale,
- les huiles de synthèse comme les alcools gras tels que l'octyl-2-dodécanol; les esters et en particulier les esters d'acides gras, et notamment les esters ayant un nombre total d'atomes de carbone choisi entre 12 et 80, de préférence entre 16 et 50; les silicones phénylées, et notamment les phényltriméthicones, les diphényl-diméthicones, les polyméthylphénylsiloxanes.
L'homme du métier sait, sur la base de ses connaissances, déterminer par de simples essais de routine les huiles solvantes de l'alkyléther de polysaccharide.

Des huiles complémentaires, non solvantes de l'alkyléther de polysaccharide, peuvent en outre être ajoutées dans la phase huileuse de l'émulsion. Comme huile complémentaire, on peut notamment citer les résines et les gommes de silicone liquides à température ambiante, les huiles hydrocarbonées partiellement fluorées, les huiles perfluorées, les huiles siliconées exemptes de groupements aromatiques telles que les polysiloxanes linéaires ou ramifiés comme les polydiméthylpolysiloxanes, les polyéthylméthylpolysiloxanes, polyalkylméthylsiloxanes et les polysiloxanes cycliques tels que octaméthylcyclotétrasiloxane, décaméthylcyclopentasiloxane ou leurs mélanges; les huiles de silicones fluorées ; les polysiloxanes fonctionnalisés par une ou plusieurs fonctions hydroxyles et/ou un ou plusieurs groupements polyéthers tels que les diméthicones copolyols; les hydrocarbures linéaires ou ramifiés, comme l'huile de vaseline, l'isohexadécane, l'isododécane.

Les huiles solvantes de l'alkyléther de polysaccharide peuvent représenter 75 à 95% en poids du poids total de la phase huileuse de l'émulsion. Les huiles complémentaires peuvent être ajoutées en une quantité pouvant aller de 0 à 75% en poids, par rapport au poids total de la phase huileuse, de préférence de 0 à 50% en poids.

L'émulsion selon l'invention peut comprendre 1-50% en poids de phase grasse, de préférence 5-30 % en poids, et plus préférentiellement 10-20% en poids de phase grasse.

De façon connue, l'émulsion selon l'invention peut éventuellement contenir une faible quantité d'un tensioactif notamment H/E, bien que cela ne soit pas nécessaire pour obtenir une émulsion stable. La quantité de tensioactif peut représenter de 0,1 à 3% et de préférence de 0,1 à 2 % du poids total de l'émulsion. Avantageusement, l'émulsion ne comprend pas de tensioactif.

L'émulsion selon l'invention peut être notamment utilisée dans les domaines cosmétique et/ou dermatologique. Elle peut être utilisée telle quelle, et donc constituer elle-même une composition cosmétique ou dermatologique; elle peut également être incorporée dans une composition cosmétique ou dermatologique plus élaborée.

Les compositions selon l'invention, constituées par ou comprenant ladite émulsion, contiennent un milieu cosmétiquement ou dermatologiquement acceptable, c'est-à-dire un milieu compatible avec toutes les matières kératiniques telles que la peau, les ongles, les muqueuses et les cheveux ou toute autre zone cutanée du corps. Ce milieu peut comprendre, de manière connue en soi, les constituants usuellement employés dans le domaine d'application considéré.
En particulier, ces compositions peuvent comprendre :
- des cires choisies parmi les cires animales, fossiles, végétales, minérales ou de synthèse connues en soi, telles que les cires de paraffine, les cires de polyéthylène, les cires de Carnauba, de Candellila; les cires d'abeilles; la cire microcristalline les cires de silicone.
- des actifs cosmétiques et/ou dermatologiques, hydrophiles ou lipophiles, tels que les adoucissants, les antioxydants, les opacifiants, les émollients, les hydroxyacides, les agents anti-mousse, les hydratants, les vitamines, les parfums, les conservateurs, les colorants, les séquestrants, les polymères, les propulseurs, les agents alcalinisants ou acidifiants, des filtres UV, des céramides; des agents anti-radicaux libres; des agents amincissants ; des bactéricides; des antipelliculaires; des complexants; des absorbeurs d'odeur;
- des matières pulvérulentes telles que des charges, des pigments et/ou des nacres.
Ces adjuvants, selon leur nature, peuvent être introduits dans la phase grasse ou dans la phase aqueuse. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

L'émulsion et la composition selon l'invention peuvent être préparées selon les techniques connues de l'homme du métier.
En particulier, l'émulsion peut être préparée en solubilisant l'alkyléther de polysaccharide dans la phase huileuse chauffée à 60-80°C, puis en solubilisant l'épaississant hydrophile dans la phase aqueuse chauffée à 60-80°C, et en dispersant ladite phase grasse dans ladite phase aqueuse sous agitation.

Les compositions selon l'invention peuvent se présenter sous forme de suspension ou de dispersion dans des corps gras; sous forme de dispersion vésiculaire non ionique; sous forme d'émulsion simple ou complexe (H/E, E/H, H/E/H ou E/H/E), de préférence de type huile-dans-eau; une crème, un lait, un gel, une pommade, une mousse aérosol ou un spray; un sérum, une pâte.
Elles trouvent en particulier une application dans un grand nombre de traitements cosmétique ou dermatologique de la peau, des cheveux, des cils, des sourcils, des ongles, des muqueuses, du cuir chevelu; en particulier on peut citer le soin du visage et/ou du corps (crèmes de protection, de traitement ou de soin pour le visage, pour les mains ou pour le corps; laits corporels de protection ou de soin; lotions, gels ou mousses pour le soin de la peau et des muqueuses ou pour le nettoyage de la peau); le maquillage du visage et/ou du corps (rouge à lèvres, eye-liner, fond de teint, mascara, anti-cernes, fard à paupières ou à joues); le démaquillage; la protection solaire; le traitement dermatologique des maladies de la peau, des cheveux, des cils des sourcils, des ongles, du cuir chevelu et/ou des muqueuses.
Elles trouvent une application préférée dans des compositions, cosmétique ou dermatologique, destinées aux peaux sèches et/ou sensibles.

L'invention est illustrée plus en détail dans les exemples suivants, dans lesquels les % sont donnés en poids.

### Exemple 1 hors invention: Crème lissante contour des yeux

| *phase A* | |
|---|---|
| Poly(acide 2-acrylamido 2-méthylpropane sulfonique) réticulé (exemple A de EP815844) à 2% dans l'eau | 1 % |
| Glycérol | 5 % |
| Conservateurs | 0,3 % |
| Eau | qsp 100% |

| *phase* B | |
|---|---|
| Huile de sésame | 10% |
| Huile de ricin | 5 % |
| Cyclohexadiméthylsiloxane | 5 % |
| Ethyl guar de degré de substitution d'environ 2,5 (N-HANCE AG 200 ® de la société Aqualon) | 1 % |

L'émulsion est préparée de la manière suivante : les phases A et B sont préparées par simple mélange à chaud des constituants et homogénéisées séparément sous agitation à 70-75°C. Puis on disperse sous agitation la phase grasse B dans la phase aqueuse A.
On obtient une émulsion de texture crémeuse et onctueuse.

### Exemple 2: Crème nutritive pour peaux très sèches

| *phase A* | |
|---|---|
| Poly(acide 2-acrylamido 2-méthylpropane sulfonique) réticulé (exemple A de EP815844) à 2% dans l'eau | 1 % |
| Glycérol | 5 % |
| Conservateurs | 0,3 % |
| Eau | qsp 100% |

| *phase* B | |
|---|---|
| Huile de soja | 20 % |
| Triglycérides d'acides caprylique/caprique | 5 % |
| Cyclohexadiméthylsiloxane | 5 % |
| Ethyl guar (N-HANCE AG 50 ® de la société Aqualon) | 1,5% |

L'émulsion est préparée selon l'exemple 1. On obtient une crème de texture sèche à l'application, apportant un effet apaisant sur la peau.

### Exemple 3: Emulsion démaquillante pour peaux sèches

| *phase* A | |
|---|---|
| Poly(acide 2-acrylamido 2-méthylpropane sulfonique) réticulé (exemple A de EP815844) à 2% dans l'eau | 1 % |
| Conservateurs | 0.3 % |
| Eau | qsp 100 % |

| *phase* B | |
|---|---|
| Palmitate d'isopropyle | 10 % |
| Octanoate d'octyle | 10 % |
| Ethyl guar (N-HANCE AG 50 ® de la société Aqualon) | 1 % |

L'émulsion est préparée selon l'exemple 1. On obtient une émulsion souple, s'étalant facilement sur la peau et présentant un très bon pouvoir démaquillant.

### Exemple 4

On compare, au microscope, les émulsions A et B suivantes :

### Emulsion A:

| *phase* A | |
|---|---|
| Poly(acide 2-acrylamido 2-méthylpropane sulfonique) réticulé (exemple A de EP815844) à 2% dans l'eau | 1,5% |
| Conservateurs | 0,3 % |
| Eau | qsp 100% |

| *phase B* | |
|---|---|
| Ethyl guar (N-HANCE AG 50 ® de la société Aqualon) | 0,8 % |
| Triglycérides d'acides caprylique/caprique | 19,2 % |

### Emulsion B:

| *phase A* | |
|---|---|
| Poly(acide 2-acrylamido 2-méthylpropane sulfonique) réticulé (exemple A de EP815844) à 2% dans l'eau | 1,5 % |
| Conservateurs | 0,3 % |
| Eau | qsp 100% |

| *phase* B | |
|---|---|
| Triglycérides d'acides caprylique/caprique | 20 % |

On obtient les résultats suivants :
- l'émulsion A est fine, régulière, les bords sont nets. Après conservation à 20°C pendant 18 mois, elle est toujours stable.
- l'émulsion B est instable et se 'déphase' rapidement, le jour même de sa fabrication.

## Revendications

1. Emulsion comprenant une phase aqueuse et une phase grasse, comprenant en outre au moins un composé épaississant hydrophile, au moins un alkyléther de polysaccharide formé de deux motifs comportant au moins deux cycles osidiques différents, chaque motif comportant au moins un groupe hydroxyle substitué par une chaîne alkyle hydrocarbonée saturée, et au moins un milieu solvant de l'alkyléther de polysaccharide, ledit milieu solvant représentant de 75 à 99,9 % en poids du poids total de la phase huileuse de l'émulsion.

2. Emulsion selon la revendication 1, dans laquelle le composé épaississant hydrophile est choisi parmi :
- les polymères synthétiques;
- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose,
- le silicate de magnésium et d'aluminium;
- les épaississants inorganiques tels que les smectites, les hectorites modifiées ou non ;
- leurs mélanges.

3. Emulsion selon l'une des revendications précédentes, dans laquelle le composé épaississant hydrophile est choisi parmi :
(A) les acides polyacryliques et notamment les polymères poly(méth)acrylates de glycéryl ; les homopolymères et les copolymères d'acide acrylique, éventuellement réticulés, ou un de leurs sels,
(B) les polymères à base de polyacrylamide et notamment :
(i) une émulsion H/E comprenant 35-45 % en poids de copolymère réticulé d'acrylamide/acide 2-acrylamido 2-méthylpropane sulfonique neutralisé, 15-25 % en poids d'hydrocarbures isoparaffiniques, 3-8 % en poids de lauryléther de polyéthylèneglycol 7OE, et d'eau;
(ii) les copolymères acrylate/octyl-acrylamide ;
(iii) les polymères réticulés d'acrylamide et de chlorure de méthacryloyloxyéthyltriméthylammonium ;
(iv) les polymères réticulés d'acrylamide et d'acrylate d'ammonium ;
(v) les polymères poly(acide 2-acrylamido 2-méthylpropane sulfonique) réticulés et pratiquement ou totalement neutralisés;
(C) les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique, ces copolymères étant éventuellement réticulés.

4. Emulsion selon l'une des revendications précédentes, dans laquelle le composé épaississant hydrophile est choisi parmi les polymères poly(acide 2-acrylamido 2-méthylpropane sulfonique) réticulés et pratiquement ou totalement neutralisés, comprenant, distribués de façon aléatoire :
a) de 90 à 99,9 % en poids de motifs de formule générale (1) suivante : 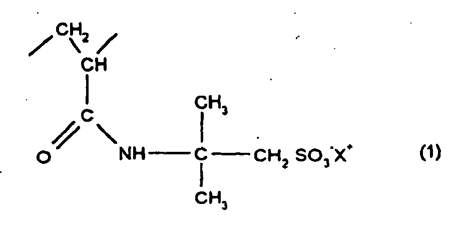 dans laquelle X⁺ désigne un cation ou un mélange de cations, au plus 10% mol des cations X⁺ pouvant être des protons H⁺ ;
b) de 0,01 à 10% en poids de motifs réticulants provenant d'au moins un monomère ayant au moins deux doubles liaisons oléfiniques ; les proportions en poids étant définis par rapport au poids total du polymère.

5. Emulsion selon l'une des revendications précédentes, dans laquelle le composé épaississant hydrophile est un copolymère acrylate/C₁₀-C₃₀-alkylacrylate.

6. Emulsion selon l'une des revendications précédentes, dans laquelle le composé épaississant hydrophile est présent à une concentration de 0,05-10 % en poids, de préférence 0,5-4 % en poids.

7. Emulsion selon l'une des revendications précédentes, dans laquelle la chaîne alkyle hydrocarbonée de l'alkyléther de polysaccharide comporte 1 à 24, de préférence de 1 à 10, mieux de 1 à 6 et plus spécialement de 1 à 3 atomes de carbone.

8. Emulsion selon l'une des revendications précédentes, dans laquelle l'alkyléther de polysaccharide a un poids moléculaire moyen en poids supérieur à 100 000, et de préférence supérieur à 200 000.

9. Emulsion selon l'une des revendications précédentes, dans laquelle l'alkyléther de polysaccharide comporte un à six, et mieux de deux à quatre groupes hydroxyle par motif, substitués par une chaîne alkyle hydrocarbonée saturée ou non.

10. Emulsion selon l'une des revendications précédentes, dans laquelle l'alkyléther de polysaccharide est un alkyléther d'une gomme, de préférence d'une gomme globalement non ionique.

11. Emulsion selon la revendication 10, dans laquelle la gomme est choisie parmi la gomme de guar; la gomme de caroube; la gomme de karaya ; la gomme adragante.

12. Emulsion selon l'une des revendications précédentes, dans laquelle l'alkyléther de polysaccharide est un galactomannane alkylé de chaîne alkyle en C₁ à C₆ et mieux en C₁ à C₃ et plus particulièrement le guar éthylé ayant un degré de substitution de 2 à 3 et notamment d'environ 2,5 à 2,9.

13. Emulsion selon l'une des revendications précédentes, dans laquelle l'alkyléther de polysaccharide est présent à une concentration de 0,1-10 % en poids, de préférence 0,5-2 % en poids.

14. Emulsion selon l'une des revendications précédentes, dans laquelle le milieu solvant de l'alkyléther de polysaccharide comprend :
- des huiles d'origine végétale comme les triglycérides liquides, par exemple les huiles de tournesol, de maïs, de soja, de jojoba, de courge, de pépins de raisin, de sésame, de noisette, d'abricot, de macadamia, de ricin; les triglycérides des acides caprylique/caprique;
- des huiles d'origine animale telle que la lanoline,
- des huiles d'origine minérale, et/ou
- des huiles de synthèse comme les alcools gras tels que l'octyl-2-dodécanol; les esters et en particulier les esters d'acides gras, et notamment les esters ayant un nombre total d'atomes de carbone choisi entre 12 et 80, de préférence entre 16 et 50; les silicones phénylées, et notamment les phényltriméthicones, les diphényldiméthicones, les polyméthylphénylsiloxanes.

15. Emulsion selon l'une des revendications précédentes, dans laquelle le milieu solvant de l'alkyléther de polysaccharide représente 75 à 95 % en poids du poids total de la phase huileuse de l'émulsion.

16. Emulsion selon l'une des revendications précédentes, se présentant sous la forme d'une émulsion huile-dans-eau, comprenant une phase grasse ou huileuse interne dispersée dans une phase externe aqueuse.

17. Emulsion selon l'une des revendications précédentes, ne comprenant pas de tensioactif.

18. Composition cosmétique ou dermatologique comprenant, dans un milieu cosmétiquement ou dermatologiquement acceptable, une émulsion telle que définie selon l'une des revendications 1 à 17.

19. Utilisation d'une émulsion telle que définie selon l'une des revendications 1 à 17, pour le traitement cosmétique de la peau, des cheveux, des cils, des sourcils, des ongles, des muqueuses, du cuir chevelu; en particulier pour le soin du visage et/ou du corps, le maquillage du visage et/ou du corps, le démaquillage; la protection solaire.

20. Utilisation d'une émulsion telle que définie selon l'une des revendications 1 à 17, pour la fabrication d'une composition dermatologique destinée au traitement de la peau, des cheveux, des cils, des sourcils, des ongles, du cuir chevelu et/ou des muqueuses.

21. Utilisation selon l'une des revendications 19 à 20, destinée aux peaux sèches et/ou sensibles.

22. Procédé de traitement non thérapeutique de la peau, des cheveux, des cils, des sourcils, des ongles, des muqueuses, du cuir chevelu, consistant à appliquer sur le support une émulsion telle que définie selon l'une des revendications 1 à 17, ou une composition telle que définie à la revendication 18.

23. Procédé de traitement non thérapeutique des peaux sèches et/ou sensibles, consistant à appliquer sur le support une émulsion telle que définie selon l'une des revendications 1 à 17, ou une composition telle que définie à la revendication 18.

24. Utilisation d'un composé épaississant hydrophile et d'un alkyléther de polysaccharide formé de deux motifs comportant au moins deux cycles osidiques différents, chaque motif comportant au moins un groupe hydroxyle substitué par une chaîne alkyle hydrocarbonée saturée, pour stabiliser une émulsion comprenant un milieu solvant dudit alkyléther de polysaccharide représentant de 75 à 99,9 % en poids du poids total de la phase huileuse de l'émulsion.

25. Utilisation selon la revendication 24, pour stabiliser une émulsion huile-dans-eau.

26. Utilisation selon l'une des revendications 24 à 25, pour stabiliser une émulsion ne comprenant pas de tensioactif.

## Claims

1. Emulsion comprising an aqueous phase and a fatty phase, additionally comprising at least one hydrophilic thickening compound, at least one polysaccharide alkyl ether formed of two units comprising at least two different glycoside rings, each unit comprising at least one hydroxyl group substituted by a saturated hydrocarbon alkyl chain, and at least one medium which is a solvent for the polysaccharide alkyl ether, said solvent medium representing 75 to 99,9 by weight of the total weight of the oily phase of the emulsion.

2. Emulsion according to Claim 1, in which the hydrophilic thickening compound is chosen from:
- synthetic polymers;
- polysaccharide biopolymers, such as xanthan gum, locust bean gum, guar gum, alginates or modified celluloses, for example hydroxyethylcellulose, methylcellulose, hydroxypropylcellulose and carboxymethylcellulose;
- magnesium aluminium silicate;
- inorganic thickeners, such as smectites or hectorites, which are or are not modified;
- their mixtures.

3. Emulsion according to either of the preceding claims, in which the hydrophilic thickening compound is chosen from:
(A) poly(acrylic acid)s and in particular poly(glyceryl (meth)acrylate) polymers; homopolymers and copolymers of acrylic acid, which are optionally crosslinked, or one of their salts,
(B) polyacrylamide-based polymers and in particular:
(i) an O/W emulsion comprising 35-45% by weight of neutralized crosslinked acrylamide/2-acrylamido-2-methylpropanesulphonic acid copolymer, 15-25% by weight of isoparaffin hydrocarbons, 3-8% by weight of polyethylene glycol 7 EO lauryl ether, and water;
(ii) acrylate/octylacrylamide copolymers;
(iii) crosslinked polymers of acrylamide and of methacryloyloxyethyltrimethylammonium chloride;
(iv) crosslinked polymers of acrylamide and of ammonium acrylate;
(v) crosslinked and virtually or completely neutralized poly(2-acrylamido-2-methylpropanesulphonic acid) polymers;
(C) copolymers composed of a predominant fraction of monomer which is monoolefinically unsaturated C₃-C₆ carboxylic acid or its anhydride and of a minor fraction of monomer which is ester comprising a fatty chain of acrylic acid, these copolymers optionally being crosslinked.

4. Emulsion according to one of the preceding claims, in which the hydrophilic thickening compound is chosen from crosslinked and virtually or completely neutralized poly(2-acrylamido-2-methylpropanesulphonic acid) polymers comprising, distributed randomly:
a) from 90 to 99.9% by weight of units of following general formula (1): 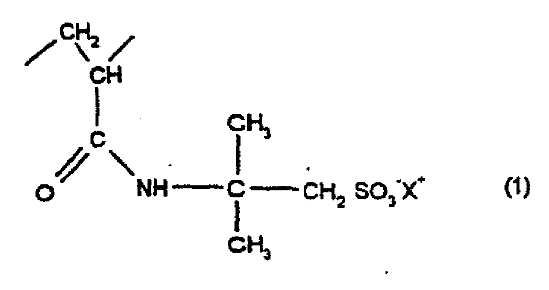 in which X⁺ denotes a cation or a mixture of cations, it being possible for at most 10 mol% of the cations X⁺ to be protons H⁺;
b) from 0.01 to 10% by weight of crosslinking units originating from at least one monomer having at least two olefinic double bonds; the proportions by weight being defined with respect to the total weight of the polymer.

5. Emulsion according to one of the preceding claims, in which the hydrophilic thickening compound is an acrylate/(C₁₀-C₃₀)alkylacrylate copolymer.

6. Emulsion according to one of the preceding claims, in which the hydrophilic thickening compound is present at a concentration of 0.05-10% by weight, preferably 0.5-4% by weight.

7. Emulsion according to one of the preceding claims, in which the hydrocarbon alkyl chain of the polysaccharide alkyl ether comprises 1 to 24, preferably from 1 to 10, better still from 1 to 6 and more especially from 1 to 3 carbon atoms.

8. Emulsion according to one of the preceding claims, in which the polysaccharide alkyl ether has a weight-average molecular weight of greater than 100 000 and preferably of greater than 200 000.

9. Emulsion according to one of the preceding claims, in which the polysaccharide alkyl ether comprises one to six and better still from two to four hydroxyl groups per unit which are substituted by a saturated or unsaturated hydrocarbon alkyl chain.

10. Emulsion according to one of the preceding claims, in which the polysaccharide alkyl ether is an alkyl ether of a gum, preferably of a gum which is nonionic overall.

11. Emulsion according to Claim 10, in which the gum is chosen from guar gum, locust bean gum, karaya gum or gum tragacanth.

12. Emulsion according to one of the preceding claims, in which the polysaccharide alkyl ether is a galactomannan alkylated with a C₁ to C₆ and better still C₁ to C₃ alkyl chain and more particularly ethylated guar gum having a degree of substitution of 2 to 3 and in particular of approximately 2.5 to 2.9.

13. Emulsion according to one of the preceding claims, in which the polysaccharide alkyl ether is present at a concentration of 0.1-10% by weight, preferably 0.5-2% by weight.

14. Emulsion according to one of the preceding claims, in which the medium which is a solvent for the polysaccharide alkyl ether comprises:
- oils of vegetable origin, such as liquid triglycerides, for example sunflower, maize, soybean, jojoba, cucumber, grape seed, sesame, hazelnut, apricot, macadamia and castor oils, or triglycerides of caprylic/capric acids;
- oils of animal origin, such as lanolin,
- oils of mineral origin, and/or
- synthetic oils, such as fatty alcohols, such as 2-octyldodecanol; esters and in particular esters of fatty acids and especially esters having a total number of carbon atoms chosen between 12 and 80, preferably between 16 and 50; or phenylated silicones, in particular phenyl trimethicones, diphenyl dimethicones or polymethylphenylsiloxanes.

15. Emulsion according to one of the preceding claims, in which the medium which is a solvent for the polysaccharide alkyl ether represents 75 to 95% by weight of the total weight of the oily phase of the emulsion.

16. Emulsion according to one of the preceding claims, which is provided in the form of an oil-in-water emulsion comprising an internal fatty or oily phase dispersed in an external aqueous phase.

17. Emulsion according to one of the preceding claims, which does not comprise surfactant.

18. Cosmetic or dermatological composition comprising, in a cosmetically or dermatologically acceptable medium, an emulsion as defined according to one of Claims 1 to 17.

19. Use of an emulsion as defined according to one of Claims 1 to 17 for the cosmetic treatment of the skin, hair, eyelashes, eyebrows, nails, mucous membranes or scalp; in particular for caring for the face and/or body, making up the face and/or body, or removing make-up; or sun protection.

20. Use of an emulsion as defined according to one of Claims 1 to 17 for the manufacture of a dermatological composition intended for the treatment of the skin, hair, eyelashes, eyebrows, nails, scalp and/or mucous membranes.

21. Use according to either of Claims 19 and 20, intended for dry and/or sensitive skin.

22. Process for the non-therapeutic treatment of the skin, hair, eyelashes, eyebrows, nails, mucous membranes or scalp, which consists in applying, to the substrate, an emulsion as defined according to one of Claims 1 to 17 or a composition as defined in Claim 18.

23. Process for the non-therapeutic treatment of dry and/or sensitive skin, which consists in applying, to the substrate, an emulsion as defined according to one of Claims 1 to 17 or a composition as defined in Claim 18.

24. Use of a hydrophilic thickening compound and of a polysaccharide alkyl ether formed of two units comprising at least two different glycoside rings, each unit comprising at least one hydroxyl group substituted by a saturated hydrocarbon alkyl chain, for stabilizing an emulsion comprising one medium which is a solvent for the said polysaccharide alkyl ether representing 75% to 99,9% by weight of the total weight of the oily phase of the emulsion.

25. Use according to Claim 24, for stabilizing an oil-in-water emulsion.

26. Use according to either of Claims 24 and 25, for stabilizing an emulsion which does not comprise surfactant.

## Patentansprüche

1. Emulsion, die eine wässrige Phase und eine Fettphase und ferner mindestens eine hydrophile verdickende Verbindung, mindestens einen Polysaccharidalkylether, der aus zwei Einheiten gebildet wird, die mindestens zwei verschiedene Zuckerringe aufweisen, wobei jede Einheit mindestens eine Hydroxygruppe enthält, die mit einer gesättigten Alkylgruppe (Kohlenwasserstoffgruppe) substituiert ist, und mindestens ein Lösungsmittelmedium für den Polysaccharidalkylether enthält, wobei das Lösungsmittelmedium 75 bis 99,9 Gew.-% des Gesamtgewichts der Ölphase der Emulsion ausmacht.

2. Emulsion nach Anspruch 1, wobei die hydrophile verdickende Verbindung ausgewählt ist unter:
- synthetischen Polymeren;
- Polysaccharidbiopolymeren, wie Xanthangummi, Johannisbrot-Kernmehl, Guargummi, Alginaten, modifizierten Cellulosen wie Hydroxymethylcellulose, Methylcellulose, Hydroxypropylcellulose und Carboxymethylcellulose;
- Magnesiumaluminiumsilikat;
- anorganischen Verdickungsmitteln, wie Smektiten, modifizierten oder nicht modifizierten Hectoriten;
- und deren Gemischen.

3. Emulsion nach einem der vorhergehenden Ansprüche, bei der die hydrophile verdickende Verbindung ausgewählt ist unter:
(A) Polyacrylsäuren und insbesondere Polyglyceryl(meth)acrylatpolymeren; Homopolymeren und Copolymeren von Acrylsäure, die gegebenenfalls vernetzt sind, oder einem ihrer Salze;
(B) Polymeren auf der Basis von Polyacrylamid und insbesondere:
(i) einer O/W-Emulsion, die 35 bis 45 Gew.-% vernetztes Copolymer Acrylamid/neutralisierte 2-Acrylamido-2-methylpropansulfonsäure, 15 bis 25 Gew.-% isoparaffinische Kohlenwasserstoffe, 3 bis 8 Gew.-% Polyethylenglykollaurylether 7 EO und Wasser enthält;
(ii) Acrylat/Oetylacrylamid-Copolymeren;
(iii) vernetzten Polymeren von Acrylamid und Methacryloyloxyethyltrimethylammoniumchlorid;
(iv) vernetzten Polymeren von Acrylamid und Ammoniumacrylat;
(v) Poly-(2-acrylamido-2-methylpropansulfonsäure)-Polymeren, die vernetzt und ganz oder teilweise neutralisiert sind;
(C) Copolymeren, die hauptsächlich aus einem monoolefinisch ungesättigten Carbonsäuremonomer mit 3 bis 6 Kohlenstoffatomen oder seinem Anhydrid und in einem geringeren Anteil einem Acrylsäureestermonomer mit Fettkette bestehen, wobei diese Copolymere gegebenenfalls vernetzt sind.

4. Emulsion nach einem der vorhergehenden Ansprüche, wobei die hydrophile verdickende Verbindung unter den vernetzten und praktisch ganz oder teilweise neutralisierten 2-(Acrylamido-2-rnethylpropansulfonsäure)-Polymeren ausgewählt ist, die zufällig verteilt enthalten:
a) 90 bis 99,9 Gew.-% Einheiten der folgenden allgemeinen Formel (1): 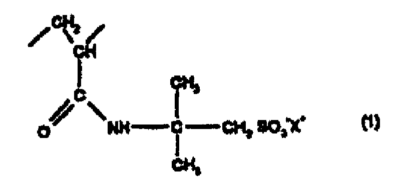 worin X⁺ ein Kation oder ein Gemisch von Kationen bedeutet, wobei höchstens 10 Mol-% der Kationen X⁺ Protonen H⁺ sein können,
b) 0,01 bis 10 Gew.-% vernetzende Einheiten, die von mindestens einem Monomer mit mindestens zwei olefinischen Doppelbindungen abgeleitet sind; wobei die Gewichtsanteile bezogen auf das Gesamtgewicht des Polymers definiert sind.

5. Emulsion nach einem der vorhergehenden Ansprüche, wobei die hydrophile verdickende Verbindung ein Acrylat/C₁₀₋₃₀-Alkylacrylat-Copolymer ist.

6. Emulsion nach einem der vorhergehenden Ansprüche, wobei die hydrophile verdickende Verbindung in einer Konzentration von 0,05 bis 10 Gew.-% und vorzugsweise 0,5 bis 4 Gew.-% enthalten ist.

7. Emulsion nach einer der vorhergehenden Ansprüche, **dadurch gekennzeichnet, dass** die gesättigte Alkylgruppe (Kohlenwasserstoffgruppe) 1 bis 24 Kohlenstoffatome, vorzugsweise 1 bis 10 Kohlenstoffatome, besser 1 bis 6 Kohlenstoffatome und speziell 1 bis 3 Kohlenstoffatome aufweist.

8. Emulsion nach einem der vorhergehenden Ansprüche, wobei der Polysaccharidalkylether ein gewichtsmittleres Molekulargewicht über 100 000 und vorzugsweise über 200 000 besitzt.

9. Emulsion nach einem der vorhergehenden Ansprüche, wobei der Polysaccharidalkylether 1 bis 6 und vorzugsweise 2 bis 4 Hydroxygruppen pro Einheit aufweist, die mit einer gesättigten oder ungesättigten Alkylgruppe (Kohlenwasserstoffgruppe) substituiert sind.

10. Emulsion nach einem der vorhergehenden Ansprüche, wobei der Polysaccharidalkylether ein Alkylether eines Gummis und vorzugsweise eines insgesamt nichtionischen Gummis ist.

11. Emulsion nach einem der vorhergehenden Ansprüche, wobei das Gummi unter Guargummi, Johannesbrotkernmehl, Karayagummi und Tragant ausgewählt ist.

12. Emulsion nach einem der vorhergehenden Ansprüche, wobei es sich bei dem Polysaccharidalkylether um ein alkyliertes Galactomannan mit einer C₁₋₆-Alkylgruppe und besser noch einer C₁₋₃-Alkylgruppe handelt, insbesondere Ethylguar mit einem Substitutionsgrad von 2 bis 3 und insbesondere etwa 2,5 bis 2,9.

13. Emulsion nach einem der vorhergehenden Ansprüche, wobei der Polysaccharidalkylether in einer Konzentration von 0,1 bis 10 Gew.-% und vorzugsweise 0,5 bis 2 Gew.-% enthalten ist.

14. Emulsion nach einer der vorhergehenden Ansprüche, wobei das Lösungsmittelmedium des Polysaccharidalkylethers umfasst:
- Öle pflanzlicher Herkunft, wie flüssige Triglyceride, beispielsweise Sonnenblumenöl, Maisöl, Sojaöl, Jojobaöl, Kürbiskernöl, Traubenkernöl, Sesamöl, Haselnussöl, Aprikosenkernöl, Macadamiaöl, Ricinusöl, Triglyceride von Capryl/Caprinsäure;
- Öle tierischer Herkunft, wie Lanolin;
- Öle mineralischer Herkunft; und/oder
- synthetische Öle, wie Fettalkohole, beispielsweise 2-Octyldodecanol; Ester und insbesondere Fettsäureester und ganz besonders Ester mit einer Gesamtzahl an Kohlenstoffatomen im Bereich von 12 bis 80 und besser noch 16 bis 50; phenylierte Silicone und insbesondere Phenyltrimethicone, Diphenyldimethicon und Polymethylphenylsiloxane.

15. Emulsion nach einem der vorhergehenden Ansprüche, wobei das Lösungsmittelmedium des Polysaccharidalkylethers 75 bis 95 Gew.-% des Gesamtgewichts der Ölphase der Emulsion ausmacht.

16. Emulsion nach einem der vorhergehenden Ansprüche, die als Öl-in-Wasser-Emulsion vorliegt, die ein innere, in einer äußeren wässrigen Phase dispergierte Fettphase oder Ölphase enthält.

17. Emulsion nach einem der vorhergehenden Ansprüche, die keinen grenzflächenativen Stoff enthält.

18. Kosmetische oder dermatologische Zusammensetzung, die in einem kosmetisch oder dermatologisch akzeptablen Medium eine Emulsion nach einem der Ansprüche 1 bis 17 enthält.

19. Verwendung einer Emulsion nach einem der Ansprüche 1 bis 17 für die kosmetische Behandlung der Haut, der Haare, der Wimpern, der Augenbrauen, der Nägel, der Schleimhäute, der Kopfhaut; insbesondere für die Pflege des Gesichts und/oder des Körpers, zum Abschminken; für den Sonnenschutz.

20. Verwendung einer Emulsion nach einem der Ansprüche 1 bis 17 für die Herstellung einer dermatologischen Zusammensetzung, die für die Behandlung der Haut, der Haare, der Wimpern, der Augenbrauen, der Nägel, der Kopfhaut und/oder der Schleimhäute vorgesehen ist.

21. Verwendung nach einem der Ansprüche 19 bis 20 für trockene und/oder empfindliche Hauttypen.

22. Verfahren zur nichttherapeutischen Behandlung der Haut, der Haare, der Wimpern, der Augenbrauen, der Nägel, der Schleimhäute, der Kopfhaut, das darin besteht, auf den Träger eine Emulsion nach einem der Ansprüche 1 bis 17 oder eine Zusammensetzung nach Anspruch 18 aufzutragen.

23. Verfahren zur nichttherapeutischen Behandlung von trockener und/oder empfindlicher Haut, das darin besteht, auf den Träger eine Emulsion nach einem der Ansprüche 1 bis 17 oder eine Zusammensetzung nach Anspruch 18 aufzubringen.

24. Verwendung einer hydrophilen verdickenden Verbindung und eines Polysaccharidethers, der aus zwei Einheiten gebildet wird, die mindestens zwei verschiedene Zuekerringe aufweisen, wobei jede Einheit mindestens eine Hydroxygruppe enthält, die mit einer gesättigten Alkylgruppe (Kohlenwasserstoffgruppe) substituiert ist, für die Stabilisierung einer Emulsion, die ein Lösungsmittelmedium für den Polysaccharidalkylether enthält, das 75 bis 99,9 Gew.-% des Gesamtgewichts der Ölphase der Emulsion ausmacht.

25. Verwendung nach Anspruch 24 zur Stabilisierung einer Öl-in-Wasser-Emulsion.

26. Verwendung nach einem der Ansprüche 24 bis 25 zur Stabilisierung einer Emulsion, die keine grenzflächenaktiven Stoffe enthält.
